# EUROPEAN PATENT APPLICATION

(11) **EP 4 591 920 A1**
(43) Date of publication of application: **30.07.2025**
(21) Application number: 23868573.9
(22) Date of filing: 20.09.2023
(51) Int. Cl.: A61N 1/32, A61N 1/36, A61N 1/04, A61M 37/00, A61L 31/14, A61L 31/16, A61L 31/10

(54) **SELF-CURRENT-GENERATING SKIN-APPLICABLE PATCH COMPRISING BIODEGRADABLE METAL**

(30) Priority: 20.09.2022 KR 20220118466; 18.09.2023 KR 20230124162
(71) Applicant: Labnpeople Co.,Ltd., Yangju-si, Gyeonggi-do 11477 (KR)
(72) Inventor: CHO, Sung Youn, Uijeongbu-si Gyeonggi-do 11790 (KR); KIM, Eun Uk, Uijeongbu-si Gyeonggi-do 11651 (KR); KWAK, Yong Kwon, Uijeongbu-si Gyeonggi-do 11698 (KR)
(74) Representative: Maiwald GmbH
(86) International application number: PCT/KR2023/014211
(87) International publication number: WO 2024/063521

(57) **Abstract**

A self-current-generating skin-applicable patch according to the present invention comprises a first area in contact with the skin of a user and a second area, which is spaced apart from the first area and is in contact with the skin adjacent to the skin with which the first area is in contact, wherein the first area or the second area comprises: a first electrode layer, which includes a biodegradable metal that can be decomposed and absorbed by making contact with the skin of the user or reacting with moisture within the tissue, is provided, as a structure integrated with the first area, in the form of a thin sheet in direct contact with the skin of the user, and has at least one penetration part through which fluid can be transferred from the side opposite to a skin contact side; a second electrode layer, which is made of different materials having a standard reduction potential that differs from that of the material forming the first electrode layer, is positioned at the side opposite to the skin contact side of the first electrode layer while being insulated from the first electrode layer, and is at least partially extended so as to be electrically connected to the second area; and a support layer, which is in the form of a sheet having a thickness and is positioned at the side opposite to the skin contact side of the first electrode layer, and is made of a hydrophilic material capable of supporting an ionic solution, thus sufficient active ingredient iontophoresis and other microcurrent effects can be provided by means of a self-generated microcurrent even without a separate power source such as a battery.

## Description

### Technical Field

The present disclosure relates to a skin-applicable patch for delivering cosmetic or medicinal active ingredients into the body. More specifically, the present disclosure relates to a self-current-generating skin-applicable patch containing a biodegradable metal. The patch is configured to generate a micro-current by the oxidation-reduction potential difference between dissimilar metals, wherein the oxidation-reduction potential difference is caused by an ionic solution consisting of the active ingredients or an ionic solution added with the active ingredients just by applying the patch to the skin. The patch is also configured to provide a beneficial effect through decomposition action of the biodegradable metal at the contact site.

### Background Art

In general, in the transdermal method of supplying active ingredients into the body through the skin, the most common method is to apply a cream or gel-type product formulated with active ingredients to the skin surface. However, by this method, it is common for a significant amount of active ingredients not to be delivered into the tissue compared to the amount applied and to be wasted. In particular, when active ingredients, such as high molecular weight materials, have a large three-dimensional size, most of the active ingredients cannot be delivered into not only the epidermis of the skin but also the stratum corneum.

The most direct transdermal method to solve this problem is to administer active ingredients through injection. This method enables rapid absorption of active ingredients into the tissue and immediate effects since a drug is directly administered through needle insertion into the body. However, it is inconvenient because only an expert (difficult self-administration) is allowed to administer a drug. In addition, patient compliance is poor due to pain from needling since a needle for use is typically dozens of millimeters in length and several millimeters in diameter.

To minimize the side effects and drawbacks, the development of a drug delivery system through transdermal administration has been actively conducted.

As mentioned above, active ingredients that are absorbed through the skin are usually formulated in the form of liquid, cream, and gel. Due to the nature of the liquid, cream, and gel formulations, it is difficult to control a dosage, and there are problems with stickiness or contamination of a coating.

Therefore, a method of attaching a patch containing active ingredients to the skin to absorb the active ingredient through the skin has been used.

The method of administering active ingredients such as a drug using a patch enables the drug to be absorbed through the skin. This can prevent side effects caused by taking oral medications, such as gastrointestinal disorders and the effects of food. Additionally, the method enables to avoid pain and discomfort during needling. Thus, due to the convenience, the use of this method has been gradually increased recently.

A patch can have a composition broadly divided into a drug-impermeable backing layer preventing a drug as active ingredients from penetrating the tissue, a drug-permeable membrane with drug permeability, and an adhesive layer allowing for patch adhesion to the skin. In the composition, provided is a storage space where the drug is stored is interposed between the backing layer and the drug-permeable membrane.

Meanwhile, a patch has a major disadvantage, which is a requirement of weakening the skin's function as a permeability barrier due to a low absorption rate of the active ingredients through the skin. In general, an absorption rate of a patch is known to be approximately 20%.

Accordingly, various methods have been used to increase the skin absorption rate of active ingredients such as a drug. For example, chemical methods such as the use of a skin absorption enhancer and prodrug, and physical methods such as iontophoresis and electrophoresis are known methods.

However, even with these known methods, there are still limitations in improving the absorption rate of active ingredients to a satisfactory level. Thus, to achieve the expected effect, it is inevitable to use an excessive amount of active ingredients than the actual required amount, considering an absorption rate.

In particular, the type of skin absorption enhancer is determined by the design type of an agent to be used or the physical and chemical properties of an ingredient. Demonstrating the effect of the skin absorption enhancer requires a concentration above a certain level. However, when adding a skin absorption enhancer, it is not easy to prevent crystal formation over time. In addition, adhesive properties such as adhesion and cohesion may change unsuitable for use.

Additionally, electricity-based techniques require a patch to be equipped with batteries, electrodes, circuits, and other components. As a result, composition of a patch product becomes complicated, and the size of the patch increases. This not only makes the patch more expensive but also generates a large amount of waste after use.

The present applicant has been continuously researching and developing a technique for a micro-needle patch made of a biodegradable metal, as a transdermal active ingredient delivery system. The present applicant has disclosed techniques that increase the efficiency of transdermal delivery of active ingredients and have beneficial effects shown during a decomposition process through Korean Patent No. 2114472, titled "Micro Needle Using the Bioabsorbable Metal", and Korean Patent No. 2291392, titled "Multi Type Micro-needle".

These techniques have already provided excellent transdermal drug delivery methods through their characteristic structures, such as micro-needles that effectively penetrate the stratum corneum and epidermis without stimulating the body's pain nerves and multiple micro-needles arranged around a hole. However, the present applicant did not stop at this and further researched and developed a method by which active ingredients could be delivered deep into the skin within a short period, leading to the completion of the present disclosure.

Therefore, the present applicant proposed the present disclosure to solve the problems. Related conventional art documents include Korean Patent No. 10-1423241 "Produce micro-current patch" and Korean Patent No. 10-2390735 "Transdermal Drug Delivery Self-Generated Micro-current Patch and Manufacturing Method Thereof". The Korean Patent No. 10-1423241 only describes a composition for and effect of promoting metabolism and blood circulation in an affected area with a micro-current generated by metals of different materials and a gel-like adhesive layer. The Korean Patent No. 10-2390735 discloses a drug penetration structure using an iontophoresis method. However, disclosed techniques only present a conceptual configuration of an iontophoresis patch and do not provide a specific alternative in whether a sufficient iontophoresis effect can be achieved with self-generated power without a separate power source. Furthermore, the techniques do not present any optimized composition, such as micro-needles made of a biodegradable metal that can be decomposed in the body, and the resulting synergistic effects.

### Disclosure

### Technical Problem

To solve the problems, an objective of the present disclosure is to provide a self-current-generating skin-applicable patch containing a biodegradable metal, wherein the patch generates a micro-current without the need for a separate power source like a battery, maximizing the effect of delivering active ingredients into the body through iontophoresis, and at the same time the patch allows for achieving an micro-current effect known to relieve pain while repairing nerves, muscles, and tissues from their damage.

Another objective of the present disclosure is to provide a self-current-generating skin-applicable patch containing a biodegradable metal, by applying an electrode and skin-contacting part of the patch with a biodegradable metal of a predetermined composition according to the present disclosure, wherein the patch has a skin improvement effect and a drug delivery enhancer effect caused by decomposition products and hydrogen gas released during decomposition.

In addition, a further objective of the present disclosure is to provide a self-current-generating skin-applicable patch containing a biodegradable metal, wherein the patch not only increases permeability of dead skin cells by using a micro-needle in an area in contact with the user's skin, but also reduces a skin resistance level in the application area, greatly increasing an effect of delivering active ingredients into the body by iontophoresis.

### Technical Solution

To achieve the objectives mentioned above, a self-current-generating skin-applicable patch according to the present disclosure may include a first area in contact with an area of the user's skin and a second area being spaced apart from the first area and in contact with another area of the skin adjacent to the area of the skin where the first area is in contact, wherein the first area or the second area may contain a biodegradable metal which can be decomposed and absorbed by coming into contact with the user's skin or reacting with moisture within the tissue. The patch may include a first electrode layer, which is provided in the form of a thin sheet in direct contact with the user's skin as a structure integrated with the first area and has at least one penetration part fluid-transferable from the side opposite to a skin-contacting side, a second electrode layer, which is made of a different material having a standard reduction potential that differs from that of a material forming the first electrode layer, is positioned at the side opposite to the skin-contacting side of the first electrode layer while being insulated from the first electrode layer, and is at least partially extended so as to be electrically connected to the second area, and a support layer, which is in the form of a sheet having a thickness and is positioned at the side opposite to the skin-contacting side of the first electrode layer, and is made of a hydrophilic material capable of supporting an ionic solution.

At this time, the first area or the second area may include at least one micro-needle protruding in the direction of contact with the user's skin.

At this time, the biodegradable metal may be represented by the Formula 1 below.

[Formula 1] MgₐZn_{b}Ca_{c}X_{d}

(In Formula 1, a, b, c and d represent the weight percentages of each component, a+b+c+d = 100 weight%, a is the largest, b, c, and d satisfy the ranges of 0≤b≤5, 0≤c≤1, 0≤d≤1, and X is composed of at least one type of impurities consisting of elements other than Mg, Zn, and Ca)

At this time, the first area and the first electrode layer may contain a biodegradable metal according to Formula 1 above. The second electrode layer may contain a different material with a higher standard reduction potential than the material of the first electrode layer.

Meanwhile, the second electrode layer may contain at least one material selected from the group consisting of copper, zinc, silver, silver chloride, iron, and stainless steel.

At this time, the second area may contain a biodegradable metal according to Formula 1 and may be configured to be electrically connected to a portion extending from the second electrode layer.

Meanwhile, the second electrode layer may contain a biodegradable metal according to Formula 1. The first electrode layer may be made of a heterogeneous material with a higher standard reduction potential than the material of the second electrode layer.

At this time, the micro-needle preferably has a length of 0.02 mm or more.

Meanwhile, the support layer may be positioned in a laminated form, being interposed between the first electrode layer and the second electrode layer.

Alternatively, the first electrode layer and the second electrode layer may be laminated while being insulated from each other by an insulating layer. Herein, the second electrode layer may be provided with an opening that exposes at least a portion of the penetration part of the first electrode layer, and the support layer may cover at least a portion of the opening on the side opposite to the skin-contacting side of the first electrode layer.

At this time, the penetration part formed in the first electrode layer may have a perforation in the form of a hole. At least one micro-needle may be bent along the edge of the hole in the direction of contact with the user's skin.

Meanwhile, the second electrode layer may be applied in the form of a flexible cloth made of conductive fiber.

Meanwhile, the ionic solution may include at least one type selected from the group consisting of phosphate-buffered saline and sodium chloride aqueous solution and may further include at least one type of active ingredients for functional cosmetics and pharmaceuticals.

At this time, the active ingredients may be ionic.

Meanwhile, the support layer may be made of at least one type selected from the group consisting of pure cotton sheets, natural pulp sheets, and rayon sheets, which are dried fabric-type materials.

Furthermore, the support layer may further include a capsule that discharges the ionic solution by the user's initiating action.

Meanwhile, the first area has preferably at least twice as large an area as the second area.

Meanwhile, the first area formed integrally with the first electrode layer may have a thin sheet shape and a thickness of 0.07 to 0.7 mm.

### Advantageous Effects

The self-current-generating skin-applicable patch containing a biodegradable metal according to the present disclosure has the following effects due to the above-described configuration.

First, even in a configuration without a separate power source like a battery, iontophoresis and other micro-current effects can be achieved using sufficient active ingredients due to self-generated micro-current.

Second, by applying a magnesium-based biodegradable metal, represented by Formula 1 of the present disclosure and capable of being decomposed in contact with the skin or in tissues, as a material for a skin-contacting electrode, the effectiveness of delivering active ingredients can be further increased due to decomposition products that act as drug delivery enhancers.

Third, the effects of reducing wrinkles caused by by-products and hydrogen gas generated during the decomposition process, reducing skin erythema caused by inflammation, and preventing skin damage caused by the sun can be further achieved.

Fourth, by bringing microscale micro-needles to be provided for the application in the skin-contacting area of the user's skin, facilitating the delivery of active ingredients into the tissue by penetrating the stratum corneum is possible, as well as reduction in skin resistance caused by the micro-needles increases electrical force, thereby enhancing the effectiveness of drug delivery by iontophoresis.

Fifth, by appropriately disposing a different metal material, electrically connected to the first and second areas in contact with the skin, with a different standard reduction potential, thereby, appropriate products can be used depending on negative/positive ionic properties of active ingredients.

Sixth, the biodegradable metal that comes into contact with or is to be inserted into the skin causes an electrochemical reaction to release more biodegradable metal ions, thereby facilitating the recovery of a damaged skin barrier more quickly.

### Description of Drawings

Figure 1 shows a side schematic diagram of a skin-applicable patch according to a first preferred embodiment of the present disclosure;
Figure 2 shows a side schematic diagram of a skin-applicable patch according to a second preferred embodiment of the present disclosure;
Figure 3 shows a side schematic diagram of a skin-applicable patch according to a third embodiment, which is a modified example of the first embodiment of Figure 1;
Figure 4 shows a side schematic diagram of a skin-applicable patch with a built-in prefilled capsule according to a fourth preferred embodiment of the present disclosure;
Figure 5 shows a side schematic diagram showing an overall configuration of a skin-applicable patch attached to the skin by an adhesive sheet according to the present disclosure;
Figures 6a to 6h show diagrams showing the specimens corresponding to compositions of biodegradable metals applicable to the present disclosure, as plotted on an alloy phase diagram;
Figure 7 shows schematic diagrams of experiments using electrodes and the electrodes used in the experiments of the present disclosure;
Figure 8 shows diagrams showing experimental conditions for comparison using copper and SUS 301 as a positive electrode in a PBS solution, respectively;
Figure 9 shows diagrams showing experimental conditions for comparison using copper and SUS 301 as a positive electrode in a NaCl solution, respectively;
Figure 10 shows a perspective view showing a shape of a micro-needle and a penetration part that can be applied to a first area of the present disclosure;
Figures 11a to 11c show photographs of skin-applicable patch prototypes according to the present disclosure, respectively, showing cases without micro-needles, with 100 µm-long needles formed, and with 230 µm-long needles formed;
Figure 12 shows perspective views of the skin-applicable patch according to the present disclosure in a commercialized form provided with an adhesive sheet and release paper;
Figure 13 shows an enlarged view of the bottom of the patch product of Figure 12;
Figure 14 shows an exploded perspective view of a skin-applicable patch according to a sixth preferred embodiment of the present disclosure;
Figure 15 shows a photograph showing a skin attachment state of a prototype implementing the skin-applicable patch according to the embodiment of Figure 14; and
Figure 16 shows photographs of patch samples applied with a thin copper plate as a second electrode plate, a 65-µm thick Ag/AgCl coating layer on a PET film, and a 130-µm thick Ag/AgCl coating layer on a PET film from the left, respectively.

### Best Mode

Hereinafter, the advantages and features according to the configuration of the present disclosure described above, and methods for achieving them, will become clear with reference to the embodiments described in detail below along with the accompanying drawings.

However, the present disclosure is not limited to the embodiments disclosed below, but is implemented in various different forms. These embodiments are merely provided to ensure that the disclosure of the present disclosure is complete and to fully inform those skilled in the art of the scope of the invention. The present disclosure is defined only by the scope of the claims.

Primarily, an area in contact with the user's skin is made of a conductive metal material or other conductive material, and the area is made of two or more regions spaced apart from each other. Therefore, the present disclosure is based on a structure of generating a micro-current by a potential difference between two regions.

For this, a patch according to the present disclosure has the most basic battery configuration, a configuration of a voltaic battery.

In other words, a metal material (a material with a low standard reduction potential) with a high ionization tendency and a tendency to become a positive ion may be used as a negative electrode (-). In the present disclosure, the metal material is basically a magnesium-based material, the details of which will be described in detail later.

Meanwhile, a conductive material with a low ionization tendency (a material with a high standard reduction potential) may be used as a positive electrode (+). Herein, a structure that an electrolyte, which enables ion exchange may be interposed between the negative electrode and the positive electrode is provided.

A solution serving as the electrolyte is referred to as an "ionic solution" in the present disclosure. The ionic solution may be provided to be supported on a support layer made of dried fabric-type materials with an ability to support the solution, such as pure cotton sheets, natural pulp sheets, and rayon sheets.

That means a metal of a negative electrode plate is ionized into positive ions by the ionic solution supported on the support layer, emitting electrons. In the meanwhile, the emitted electrons function to generate a micro-current in a process of moving to a positive electrode plate via human tissue. The micro-current generated at this time acts as an electrolyte and at the same time allows active ingredients in the ionic solution containing the active ingredients to be delivered quickly and deeply into the subcutaneous tissue.

The active ingredients may penetrate or diffuse into the subcutaneous tissue during penetration and diffusion processes of the ionic solution or may itself have negative or positive ionic properties, thereby facilitating movement by direct electrical force.

In other words, when active ingredients with negative ionicity are supplied while a metal of the negative electrode is in contact with the skin, or active ingredients with positive ionicity are supplied while a metal of the positive electrode is in contact with the skin, delivery and diffusion effect of the active ingredients by electrical force may be maximized. To this end, the present disclosure largely illustrates two types of embodiments and modifications thereof and proposes additional embodiments.

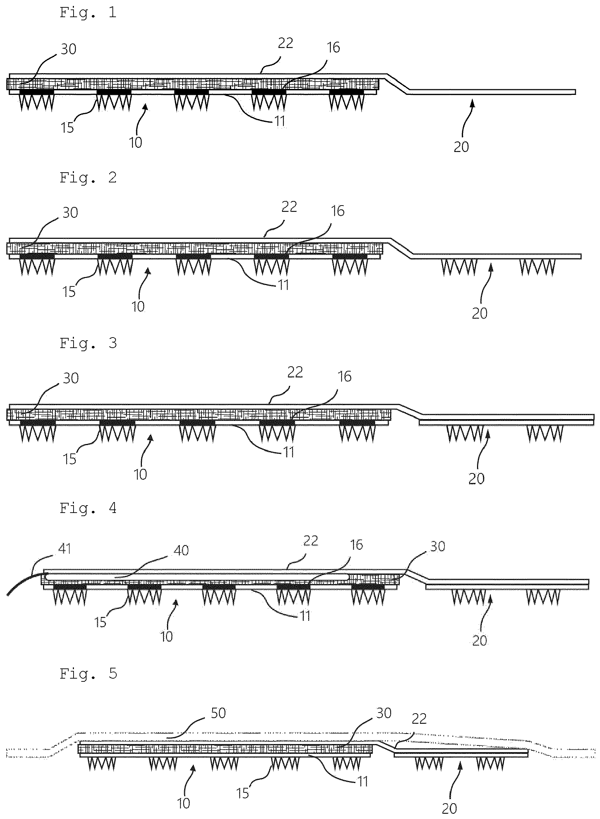

Figure 1 shows a side schematic diagram of a self-current-generating skin-applicable patch containing a biodegradable metal according to a first preferred embodiment of the present disclosure. The patch, in its configuration, may have a first area 10 that comes into contact with an area of the user's skin. A first electrode layer 11, which serves as a negative electrode, may have a magnesium-based composition represented by Formula 1 according to the present disclosure, which will be described later. A second electrode layer 22, in contact with a support layer 30 interposed between itself and the first electrode layer 11, may be made of a material with a lower ionization tendency and a higher standard reduction potential than the first electrode layer 11, such as copper, zinc, silver, silver chloride, iron, and stainless steel.

In this state, a first area 10 and a second area 20 extending from the second electrode layer 22 are in contact with the user's skin, respectively. An ionic solution serving as an electrolyte is supported on the support layer 30 interposed between the first electrode layer 11 and the second electrode layer 22, wherein the first electrode layer 11 and the second electrode layer 22 are electrically insulated by the support layer 30. When this occurs, the metal of the first electrode layer becomes ionized, resulting in the electron movement through the skin, that is, a generation of an electric current.

At this time, when the active ingredients contained in the ionic solution supported on the support layer 30 have negative ionicity, the delivery and diffusion of the active ingredients supplied to the skin under the first area 10 through a penetration part 16 may occur more effectively through iontophoresis.

Conversely, when the active ingredients contained in the ionic solution have positive ionicity, it is preferable that a difference in standard reduction potential between the first electrode layer 11 and the second electrode layer 22 be opposite to that of the first embodiment.

Figure 2 shows a side schematic diagram of a self-current-generating skin-applicable patch containing a biodegradable metal according to a second preferred embodiment of the present disclosure. Unlike the first embodiment, the first electrode layer 11 constituting the first area 10 in contact with an area of the user's skin serves as a positive electrode. The first electrode layer 11 may be made of a material, such as copper, zinc, silver, silver chloride, iron, and stainless steel, with a lower ionization tendency and a higher standard reduction potential than the second electrode layer 22 with a magnesium-based composition represented by Formula 1 according to the present disclosure, which will be described later.

In this state, when the active ingredients contained in the ionic solution supported on the support layer 30 have positive ionicity, the delivery and diffusion of the active ingredients supplied to the skin under the first area 10 through the penetration part 16 by iontophoresis may occur much more effectively than in the case of active ingredients with electrically neutral or negative ionic properties.

To clearly distinguish between these two types, the present disclosure designates the first embodiment as a basic type and the second embodiment as a reverse type.

As a modified form of the first embodiment, a patch, shown in Figure 3, may be provided.

In the first embodiment as a basic type, the second area 20 has a simple structure in which a portion extending from the second electrode layer 22 comes into direct contact with the skin. Due to that, in this embodiment, the second area 20 simply serves as an electrode for iontophoresis and micro-current.

However, even for the basic type, when a biodegradable metal with a magnesium-based composition according to Formula 1, which will be described later, is bonded to form the second area 20 in the skin-contacting direction of the second electrode layer 22 serving as a positive electrode, as in the third embodiment shown in Figure 3, the advantage is that the beneficial effects that may be obtained from the metal decomposition process may be obtained across the entire area of the patch.

Meanwhile, the structure of the self-current-generating skin-applicable patch containing a biodegradable metal according to the fourth embodiment of the present disclosure shown in Figure 4 will be described.

As described above, the support layer 30 of the present disclosure may be provided to supply an ionic solution containing active ingredients through the penetration part 16 or a side portion of the first area 10, without any ionic solution being supported.

In other words, in this case, the patch according to the present disclosure and an ampoule containing the active ingredients are provided separately in the form of a set, and the user may absorb the solution in the ampoule into the support layer 30 immediately before applying the ampoule to the skin.

However, to avoid this inconvenience in use, the patch according to the fourth embodiment, which is a modified example of the third embodiment, the basic type, may be provided with a separate capsule 40 contained within or located adjacent to the support layer 30, and this capsule 40 may have a structure of storing an ionic solution containing active ingredients.

In this case, the user may cause the ionic solution in the capsule 40 to be discharged into the support layer 30 by pulling a handle 41 connected to the capsule 40.

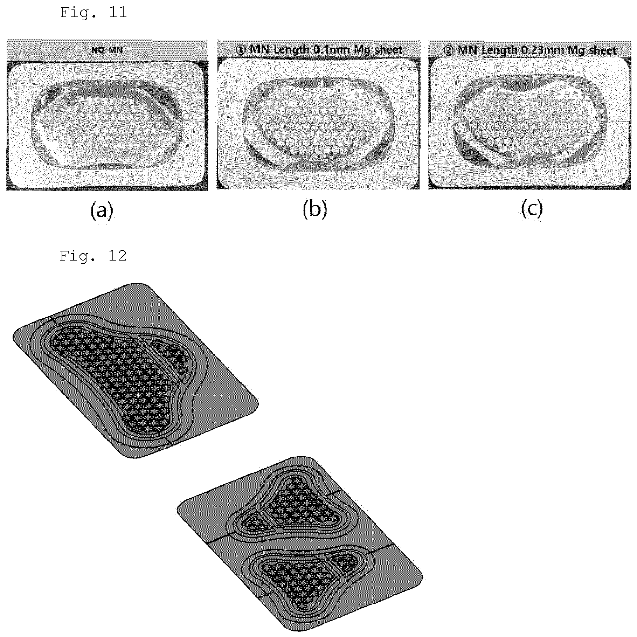

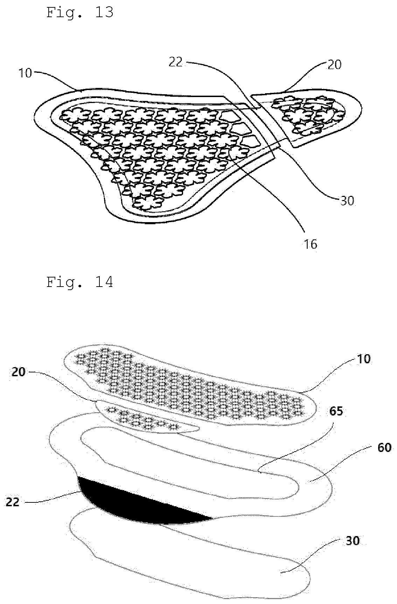

The patch according to the present disclosure illustrated in the first to fourth embodiments may be attached in close contact with the user's skin by an adhesive sheet 50, as shown in Figure 5. The patch may be provided in a commercialized form, as provided with an adhesive sheet 50 and release paper as shown in Figures 12 and 13.

Figure 13 is an enlarged view of the bottom of the patch product of Figure 12, describing the bottom of the patch product in a transparent state so that a positional relationship between each component may be identified. A first area 10, which has a large area and a plurality of penetrating holes 16, is formed as the first electrode layer 11. A portion of the second electrode layer 22 in contact with the support layer 30 interposed between itself and the first electrode layer 11 extends to the right from the position overlapping the first area 10. Therefore, the structure may be confirmed to include an extension part bonded to a thin micro-needle plate corresponding to the second area 20, which has a smaller area than the first area 10.

Now at this point, the biodegradable metal according to the present disclosure that may be applied to the first electrode layer 11 and the first area 10 in the first embodiment, the second electrode layer 22 and the second area 10 in the second embodiment, and the first electrode layer 11, first area 10, and second area 20 in the third embodiment will be described in detail.

The biodegradable metal, according to the present disclosure, may be defined by Formula 1 below.

[Formula 1] MgₐZn_{b}Ca_{c}X_{d}

In Formula 1, a, b, c, and d are the weight percentage of each component, a+b+c+d = 100% by weight, a is the largest, and therefore a has a value greater than 0 and 100 or less. Meanwhile, b, c, and d satisfy the ranges of 0≤b≤5, 0≤c≤1, 0≤d≤1, and X may be composed of at least one type of impurities consisting of elements other than Mg, Zn, and Ca.

That means that the biodegradable metal according to the present disclosure includes a metal that can be decomposed by reacting with water in vivo, but specifically includes magnesium as a main component. The biodegradable metal may include zinc and calcium mixed in an appropriate ratio to provide harmless and beneficial effects to the human body, and the biodegradable metal may be selected from a metal in various alloy phases or a metal with a composition having a different decomposition rate.

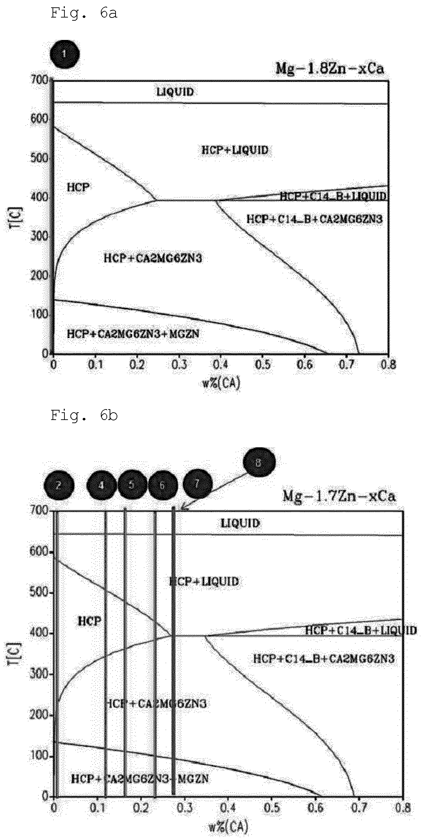

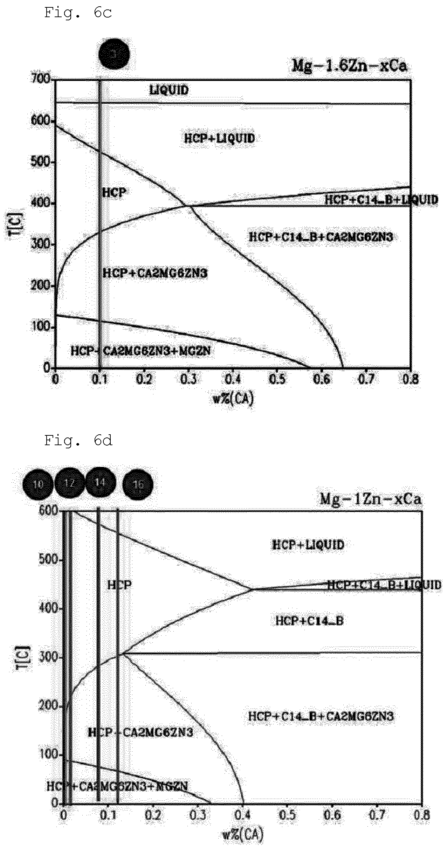

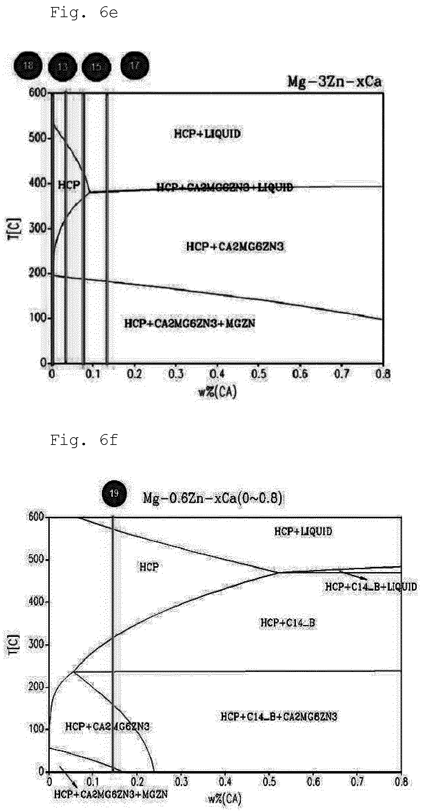

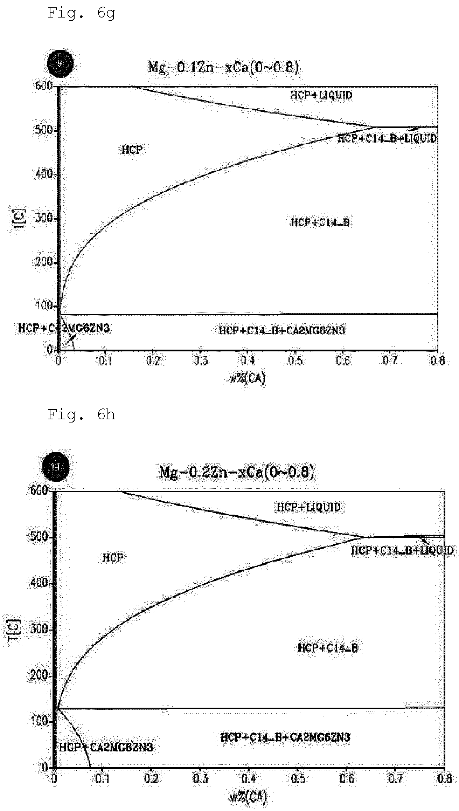

Figures 6a to 6h show internal phases of the alloys depending on the contents of magnesium, zinc, and calcium at a predetermined temperature. Depending on these contents, the internal phases of the alloys exist in the states of alpha magnesium phase (HCP), ternary phase (Ca₂Mg₆Zn₃), Mg₂Ca(C14_b), and MgZn. Among these, the Mg₂Ca phase serves to increase strength of an alloy, but it enables formation of a galvanic circuit, increasing a decomposition rate. The MgZn phase is also known to induce micro-galvanism within an alloy and increase a decomposition rate. That is, as a decomposition rate increases, an ionization degree increases compared to pure Mg, so it is expected that an electromotive force of an alloy by a difference in a standard reduction potential between the alloy and a positive electrode layer will also increase.

Taking specimen 3 in Figure 6c as an example, as an alloy that exists in a liquid state at a casting temperature of about 650°C or higher is cooled, the alloy goes through a "zone where alpha magnesium phase (HCP) + liquid phase coexists", a zone where "alpha magnesium phase (HCP)" is created, and "a zone where alpha magnesium phase (HCP) and ternary phase (Ca₂Mg₆Zn₃) phase are created". At this time, the structure of the alloy is confirmed to be formed in the alpha magnesium phase (HCP) and the ternary phase (Ca₂Mg₆Zn₃), and the formation of the MgZn phase at the bottom of the alloy may be prevented. When the MgZn phase is intentionally created with an alloy with the composition of Specimen 3, the prepared alloy is required to be heat-treated for a long time at a temperature of about 120°C or lower. Meanwhile, in a typical casting furnace, the MgZn phase is not created because an alloy is taken out of the casting furnace and rapidly cooled before it is cooled to the temperature at which the MgZn phase is formed.

Meanwhile, the impurities such as Ce, Mn, Al, Pb, Fe, Ni, Zr, Cu, Th, Be, Cd, Sn, P, Si, La, Sr, Pr, Na, and Y are introduced from crucibles during the preparation process. Herein, it is preferable that the total amount of the impurities is 1% by weight or less.

In the present disclosure, as is commonly known, the biodegradable metal may be prepared by melting and mixing magnesium, calcium, and zinc and then molding them.

The melting can be performed in an inert gas atmosphere such as argon (Ar), which does not react with magnesium, calcium, and zinc, or in a vacuum atmosphere. Various methods may be used, including a resistive heating method that generates heat by applying electricity to a resistor, an induction heating method by passing current through an induction coil, or a method using a laser or focused light.

The molding may involve, but is not limited to, implementing cooling methods, extrusion methods, and metal processing methods. The cooling methods may be used for the purpose of enhancing the mechanical strength of magnesium alloy. In more detail, a method of immersing a crucible containing molten magnesium in water may be used. Additionally, a cooling method of spraying molten magnesium using an inert gas such as argon may be used. In the spray cooling method, cooling progresses at a much higher rate, and very fine structures may appear. However, caution should be taken when casting magnesium in small sizes since multiple pores may be formed inside.

Using these processing methods, a magnesium-based alloy may be prepared with a composition and crystalline phase suitable for generating the desired level of electromotive force. For example, as a way to further increase electromotive force while using the same electrode, a galvanic circuit may be configured to accelerate a decomposition rate. In addition, the formation of an alloy in a high-strength Mg₂Ca phase may be promoted.

### Mode for Disclosure

### [Preparation of Biodegradable Metal Specimens]

Calcium, zinc, and magnesium with the composition shown in Table 1 below were charged into a stainless steel crucible (SUS 410) (or a carbon crucible) with a 50-mm internal diameter . Next, argon gas was flowed around the crucible to prevent the calcium, zinc, and magnesium in the crucible from coming into contact with air. With that, using a resistance heating furnace, the crucible temperature was raised from about 700°C to 750°C to melt calcium, zinc, and magnesium. The material in the crucible was stirred so that the molten calcium, zinc, and magnesium could be well mixed. The completely molten magnesium alloy was cooled to prepare a solid magnesium alloy. Additionally, during cooling, the crucible was immersed in water to rapidly cool the molten magnesium alloy for the purpose of improving mechanical strength of magnesium.

Specimens were prepared by extruding the solid magnesium alloy. Herein, an extrusion temperature was 400°C. The cross-sectional area reduction ratio (extrusion ratio) before and after extrusion was set at 40:1. Each composition of the prepared specimens was evaluated using a metal component analysis device (SPECTRO Maxx). The results are shown in Figures 6 (a) to 6 (h) and Table 1.

**[Table 1]**

| Mg alloy | Plan | | Actual input | | Measurement result | |
|---|---|---|---|---|---|---|
| | Ca | Zn | Ca | Zn | Ca | Zn |
| | Conc | Conc | Conc | Conc | Conc | Conc |
| | % | % | % | % | % | % |
| 1 | 0.00 | 1.60 | 0.00 | 1.60 | <0.0001 | 1.84 |
| 2 | 0.05 | 1.60 | 0.06 | 1.60 | 0.040 | 1.73 |
| 3 | 0.10 | 1.60 | 0.11 | 1.60 | 0.097 | 1.64 |
| 4 | 0.15 | 1.60 | 0.17 | 1.60 | 0.13 | 1.74 |
| 5 | 0.20 | 1.60 | 0.22 | 1.60 | 0.15 | 1.69 |
| 6 | 0.25 | 1.60 | 0.28 | 1.60 | 0.22 | 1.75 |
| 7 | 0.30 | 1.60 | 0.33 | 1.60 | 0.26 | 1.71 |
| 8 | 0.35 | 1.60 | 0.39 | 1.60 | 0.26 | 1.76 |
| 9 | 0.01 | 0.15 | 0.01 | 0.15 | 0.009 | 0.013 |
| 10 | 0.00 | 1.00 | 0.00 | 1.00 | 0.001 | 1.04 |
| 11 | 0.02 | 0.25 | 0.02 | 0.25 | 0.007 | 0.20 |
| 12 | 0.05 | 1.00 | 0.06 | 1.00 | 0.080 | 1.17 |
| 13 | 0.05 | 3.00 | 0.06 | 3.00 | 0.035 | 3.08 |
| 14 | 0.10 | 1.00 | 0.11 | 1.00 | 0.071 | 1.14 |
| 15 | 0.10 | 3.00 | 0.11 | 3.00 | 0.079 | 3.23 |
| 16 | 0.15 | 1.00 | 0.17 | 1.00 | 0.13 | 1.12 |
| 17 | 0.15 | 3.00 | 0.17 | 3.00 | 0.11 | 3.13 |
| 18 | 0.00 | 3.00 | 0.00 | 3.00 | 0.0006 | 3.15 |
| 19 | 0.16 | 0.55 | 0.18 | 0.55 | 0.14 | 0.63 |

Afterward, the prepared specimens were placed in a heating furnace and heat-treated for an additional 24 hours at a temperature of 450°C. As a result, heat-treated biodegradable metal specimens were prepared.

### [Evaluation of Decomposition Characteristics of Biodegradable Metals]

Some samples from the heat-treated biodegradable metal specimens prepared as above were extracted, and their decomposition characteristics were evaluated. For the evaluation of decomposition characteristics, considering the fact that hydrogen gas is characterized to be released when alkaline earth metals are decomposed, the amount of hydrogen gas released during decomposition was measured using an eudiometer in a PBS solution set at a temperature of 37°C. The results are shown in Table 4.

**[Table 2]**

| Division | Measurement cycle (h) | 0.5 | 1 | 2 | 4 | 8 | 24 | 48 | 72 | 96 | 120 | 144 | 168 | Hydrogen gas generation per hour |
|---|---|---|---|---|---|---|---|---|---|---|---|---|---|---|
| Commercial biodegradable alloy material 5Ca-1Zn | | 0.2 3 | 0.3 8 | 0.5 1 | 0.7 9 | 1.3 0 | 2.2 4 | 3.9 0 | 5.4 1 | 6.2 2 | 7.4 3 | 7.8 8 | 8.5 2 | 0.27 |
| 2 | #1 | 0.0 2 | 0.0 8 | 0.1 1 | 0.1 9 | 0.2 8 | 0.4 9 | 0.7 0 | 0.8 9 | 1.0 9 | 1.2 8 | 1.5 6 | 2.3 2 | 0.05 |
| | #2 | 0.02 | 0.06 | 0.09 | 0.15 | 0.19 | 0.26 | 0.45 | 0.51 | 0.60 | 0.75 | 0.75 | 0.79 | 0.03 |
| | #3 | 0.0 2 | 0.0 2 | 0.0 6 | 0.0 9 | 0.1 9 | 0.3 4 | 0.4 9 | 0.5 8 | 0.7 2 | 0.8 7 | 0.9 4 | 1.0 9 | 0.03 |
| 3 | #1 | 0.0 8 | 0.1 3 | 0.1 7 | 0.2 3 | 0.3 2 | 0.5 8 | 0.8 9 | 1.1 5 | 1.3 9 | 1.7 9 | 2.0 4 | 2.3 2 | 0.07 |
| | #2 | 0.0 8 | 0.1 3 | 0.1 9 | 0.2 8 | 0.3 8 | 0.4 5 | 0.5 3 | 0.5 8 | 0.6 8 | 0.9 0 | 1.1 3 | 1.3 6 | 0.04 |
| | #3 | 0.0 4 | 0.0 9 | 0.1 3 | 0.2 1 | 0.2 8 | 0.5 1 | 0.7 0 | 0.9 0 | 1.2 3 | 1.6 6 | 2.0 0 | 2.3 2 | 0.06 |
| 4 | #1 | 0.0 2 | 0.0 9 | 0.1 3 | 0.1 9 | 0.2 5 | 0.4 1 | 0.7 0 | 1.0 0 | 1.2 8 | 1.6 8 | 1.9 8 | 2.3 9 | 0.06 |
| | #2 | 0.0 2 | 0.0 9 | 0.1 3 | 0.2 1 | 0.3 2 | 0.6 2 | 1.0 2 | 1.3 6 | 1.7 0 | 2.2 1 | 2.3 2 | 2.5 1 | 0.07 |
| | #3 | 0.0 8 | 0.2 3 | 0.3 2 | 0.4 0 | 0.4 7 | 0.8 9 | 0.9 6 | 1.1 7 | 1.3 8 | 1.5 1 | 1.7 3 | 1.8 5 | 0.07 |
| 6 | #1 | 0.0 6 | 0.1 3 | 0.1 9 | 0.3 0 | 0.5 1 | 1.0 9 | 1.6 8 | 2.0 4 | 2.2 6 | 2.7 1 | 2.9 2 | 3.3 0 | 0.10 |
| | #2 | 0.0 6 | 0.0 9 | 0.1 7 | 0.2 6 | 0.3 8 | 0.8 1 | 1.2 4 | 1.4 1 | 1.5 1 | 1.6 6 | 1.8 3 | 2.1 7 | 0.07 |
| | #3 | 0.0 9 | 0.0 8 | 0.0 9 | 0.1 5 | 0.2 3 | 0.3 4 | 0.6 0 | 0.7 9 | 1.0 0 | 1.1 9 | 1.2 4 | 1.4 3 | 0.04 |
| 12 | #1 | 0.0 6 | 0.0 9 | 0.1 5 | 0.2 5 | 0.3 8 | 0.7 7 | 1.2 1 | 1.6 2 | 1.9 6 | 2.4 3 | 2.5 1 | 2.6 8 | 0.08 |
| | #2 | 0.0 2 | 0.0 6 | 0.0 9 | 0.1 7 | 0.2 3 | 0.3 8 | 0.5 5 | 0.6 8 | 0.7 9 | 0.9 0 | 0.9 8 | 1.0 4 | 0.04 |
| | #3 | 0.0 2 | 0.0 8 | 0.0 9 | 0.1 7 | 0.2 5 | 0.6 2 | 1.1 3 | 1.5 3 | 1.8 5 | 2.3 4 | 2.4 5 | 2.7 0 | 0.08 |
| 14 | #1 | 0.0 6 | 0.1 3 | 0.1 9 | 0.2 5 | 0.3 0 | 0.3 8 | 0.5 1 | 0.6 4 | 0.8 3 | 1.2 1 | 1.2 8 | 1.3 9 | 0.04 |
| | #2 | 0.0 6 | 0.0 9 | 0.1 5 | 0.2 1 | 0.3 0 | 0.5 8 | 0.8 9 | 1.1 9 | 1.3 9 | 1.8 5 | 1.9 2 | 2.0 4 | 0.06 |
| | #3 | 0.0 6 | 0.1 1 | 0.1 7 | 0.2 1 | 0.3 4 | 1.1 5 | 1.5 1 | 2.3 7 | 2.7 7 | 3.2 8 | 3.3 4 | 3.4 3 | 0.11 |
| 16 | #1 | 0.0 6 | 0.0 9 | 0.1 7 | 0.2 5 | 0.3 6 | 0.7 0 | 1.2 1 | 1.6 4 | 1.9 4 | 2.2 6 | 2.3 4 | 2.4 9 | 0.08 |
| | #2 | 0.0 6 | 0.1 1 | 0.1 9 | 0.2 8 | 0.3 8 | 0.4 9 | 0.5 5 | 0.5 7 | 0.6 2 | 0.7 9 | 0.8 1 | 0.8 3 | 0.03 |
| | #3 | 0.0 8 | 0.0 8 | 0.1 3 | 0.1 9 | 0.2 6 | 0.3 2 | 0.4 7 | 0.6 6 | 0.8 7 | 1.1 9 | 1.2 6 | 1.2 8 | 0.04 |
| 15 | #1 | 5 | 9 | 15 | 22 | 26 | 38 | 50 | 55 | 62 | 62 | 67 | 70 | 2.86 |
| | #2 | 1 | 3 | 6 | 10 | 66 | 209 | 399 | 400 | 403 | 405 | 408 | 411 | 16.20 |
| | #3 | 3 | 5 | 10 | 14 | 16 | 28 | 100 | 112 | 124 | 131 | 142 | 147 | 4.95 |
| 17 | #1 | 3 | 4 | 6 | 7 | 8 | 100 | 136 | 185 | 232 | 279 | 332 | 399 | 10.07 |
| | #2 | 1 | 5 | 10 | 12 | 13 | 21 | 44 | 76 | 127 | 175 | 237 | 311 | 6.14 |
| | #3 | 3 | 6 | 7 | 9 | 10 | 100 | 117 | 138 | 168 | 191 | 221 | 291 | 7.51 |

From Table 2, by comparing the commercial biodegradable alloy materials, which are comparative examples, with the specimens prepared in the examples, it was confirmed that there was a diverse spectrum of specimens with high and low decomposition rates depending on the commercial biodegradable alloy materials. Through this, it was possible to selectively apply a magnesium-based alloy composition with an appropriate decomposition rate depending on the active ingredient and application period of the skin-applicable patch provided according to the present disclosure.

Since micro-needles 15 were made of the biodegradable metal according to the composition of Formula 1, the micro-needles 15 formed in the first area 10 or the second area 20, which was a user's skin-contacting area according to the present disclosure, differed from conventional micro-needles. Due to that, after being inserted into the subcutaneous tissue or epithelium for the administration of active ingredients, the micro-needles 15 acted as an electrode and were absorbed and decomposed at the same time to release metal ions and decomposition products into the body.

Materials used for the micro-needles of the present disclosure were biodegradable metals of the alkaline earth metal series, such as magnesium (Mg), calcium (Ca), and zinc (Zn). The materials experience a mechanism of reacting with water and releasing hydrogen gas, as shown in the equation below. Therefore, micro-needles made of the metal materials emit ions and decomposition products when absorbed and decomposed under the skin. The micro-needles might induce a wrinkle reduction effect by causing a swelling effect under the skin with hydrogen gas generated as a by-product. In addition, the micro-needles might induce an effect of reducing skin erythema caused by inflammation and prevent skin damage caused by the sun (YOON, K. S. et al., Histological study on the effect of electrolyzed reduced water-bathing on UVB radiation-induced skin injury in hairless mice, Biological and Pharmaceutical Bulletin 34, 1671-7, 2011; IGNACIO, R. M., et al., The balneotherapy effect of hydrogen reduced water on UVB-mediated skin injury in hairless mice, Molecular & Cellular Toxicology 9, 15-21, 2013).

Mg + 2H₂O → Mg(OH)₂+ H₂(gas)

Zn + 2H₂O → Zn(OH)₂+ H₂(gas)

Ca + 2H₂O -> Ca(OH)₂+ H₂(gas)

In addition, ZnO and MgCl, which were by-products produced when magnesium (Mg) and zinc (Zn) were administered into the body, might serve as drug delivery enhancers that improved drug absorption even when the ZnO and MgCl did not penetrate subcutaneously and only existed on the skin surface. Therefore, the micro-needles made of a biodegradable metal might further increase the delivery effect of active ingredients supported in the patch according to the present disclosure.

Therefore, as described above, by applying the biodegradable metals to the skin-contacting area of the skin-applicable patch according to the present disclosure, synergistic effects might be obtained including decomposition product-cased beneficial effects and effects as drug delivery enhancers.

### [Electromotive Force Generation Experiments depending on Type of Electrolyte and Type of Positive Electrode Metal]

Before this experiment, as a preliminary experiment, electromotive force generation was compared between PBS and commercially available acne ampoule. However, an electromotive force obtained with the acne ampoule was found to fall short of a criterion for any positive electrode metal. Due to that, it was confirmed that an ionic solution applicable to the present disclosure should be used in a mixture state of active ingredients and a solution with a high ionization degree as an electrolyte, or an aqueous solution of the active ingredient with a high ionization degree itself.

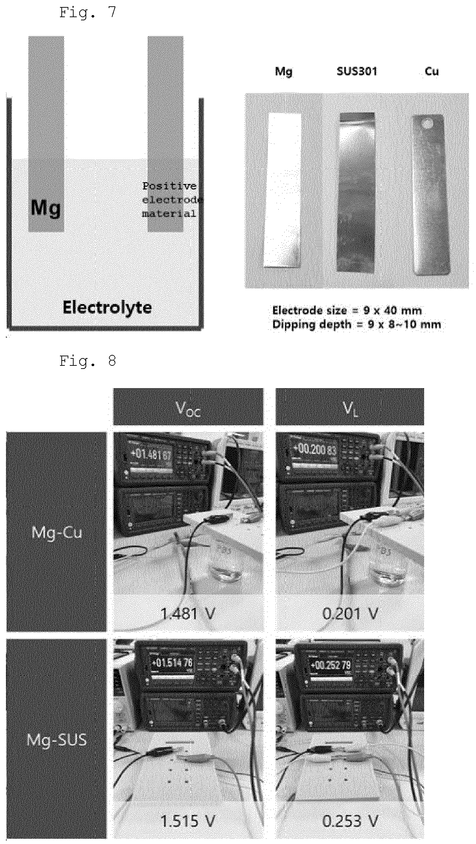

Experiments were conducted, as shown in Figure 7, wherein a pure Mg electrode containing inevitable impurities was used as a negative electrode. Herein, phosphate-buffered saline (PBS), which was a purchased commercial product, and an artificially prepared 0.9% NaCl aqueous solution were prepared as electrolytes applicable as the ionic solution of the present disclosure. SUS 301, a type of stainless steel, and copper were used as metals for a positive electrode.

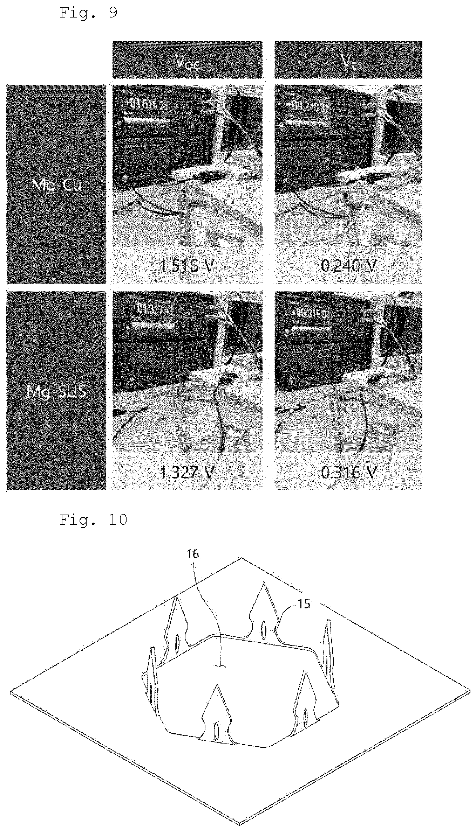

These experiments were conducted as shown in the photographs in Figures 8 and 9.

Figure 8 shows experimental conditions for comparison using copper and SUS 301 as a positive electrode in a PBS solution, respectively.

Figure 9 shows experimental conditions for comparison using copper and SUS 301 as a positive electrode in a NaCl solution, respectively.

The results are shown in Table 3 below.

**[Table 3]**

| No. | Electrodes | Electrolyte | V_{oc} [V] | V_{L} [V] | I_{L} [mA] | P_{L} [mW] |
|---|---|---|---|---|---|---|
| 1 | Mg-Cu | PBS | 1.482 | 0.201 | 0.402 | 0.081 |
| 2 | Mg-Cu | NaCl | 1.516 | 0.240 | 0.480 | 0.115 |
| 3 | Mg-SUS | PBS | 1.515 | 0.253 | 0.506 | 0.128 |
| 4 | Mg-SUS | NaCl | 1.327 | 0.316 | 0.632 | 0.200 |

A resistance of 500 Ω was used to measure the operating voltage and current along with the open circuit voltage V_{oc}. As a result, it was confirmed that an artificially prepared 0.9% NaCl aqueous solution was more advantageous than PBS as an electrolyte to be included in the ionic solution. This is presumed to be because there might be fewer interfering factors with ion movement in the aqueous solution.

Meanwhile, matching for the positive electrode showed that the Mg-SUS 301 combination was overall more advantageous in terms of electromotive force. However, it was predicted that the Mg-Cu combination would also be sufficient to be applied to the patch of the present disclosure. In human application experiments to be described later, copper was more advantageous in terms of ductility related to skin adhesion and attachment, so copper could be appropriately selected depending on the structure of a patch to be prepared.

From the experiments, it was confirmed that the ionic solution according to the present disclosure preferably included an NaCl aqueous solution and other ionic aqueous solutions with similar ionization degrees. In this regard, an ionic solution according to the present disclosure could be provided by using active ingredients solution having a comparable self-ionization degree or by mixing the above-described good electrolyte solutions with active ingredients.

There was no limitation as to the active ingredients as long as the active ingredients were ingredients that might produce effects as functional cosmetic or pharmaceutical. When the active ingredients had ionic properties, it was more preferable.

For example, the functional cosmetics were defined as follows.

- Functional cosmetics that help whiten the skin
- Functional cosmetics that help reduce skin wrinkles
- Functional cosmetics that help protect the skin from ultraviolet rays
- Functional cosmetics that help change hair color (including decolorization and bleaching)
- Functional cosmetics that help remove body hair
- Functional cosmetics that help alleviate acne-prone skin

In particular, with regard to the functions of skin whitening (brightening skin tone) and reducing skin wrinkles, and alleviating acne-prone skin, the applicant of the present disclosure has already published Korean patent No. 2194089, titled "Flexible Metal Patch having Anti-oxidant Activity and Whitening Effects and Usage" and Korean patent No. 2310566, titled "Patch for Reducing and Preventing Acne". Through the publication, it has been disclosed that the biodegradable metal according to the present disclosure exhibited the functional effects by themselves without the use of separate active ingredients. Therefore, higher synergistic effects might be expected when the skin-applicable patch containing a biodegradable metal according to the present disclosure was used with active ingredients for this purpose.

Meanwhile, as shown in Figure 10, the he micro-needles 15 and the penetration part 16 applied to the first area 10 according to the present disclosure might have a shape in which the penetration part 16 was formed in the shape of a polygonal or closed curve hole, and at least one micro-needle 15 was arranged at the edge of the penetration part 16.

The advantage of this structure was that in the preparation of a micro-needle patch, the micro-needles and the penetration part were formed simultaneously on a plane, and then the micro-needles were bent using a jig so that a patch with a plurality of micro-needles might be easily provided. In addition, the advantage of this structure was that the micro-needles were arranged around the wide penetration part in use, so active ingredients supplied from the upper part of the penetration part 16 might be easily delivered downward through the penetration part 15. Moreover, the active ingredients might be simultaneously delivered to the inside of the skin along the insertion path of the micro-needles.

In other words, in the case of the patch according to the present disclosure, active ingredients in the ionic solution supported on the support layer might be delivered to the inside of the skin through the process described above and then quickly diffused under electrical force. Thus, the structure of the micro-needless 15 and the penetration part 16 might be applied in a more optimized structure to the present disclosure.

Meanwhile, in addition to the beneficial effects generated by the micro-needless 15 according to the present disclosure, as obtained when the micro-needles were inserted into the skin and decomposed, the micro-needles 15, according to the main objectives of the present disclosure, generated a higher electromotive force, thereby playing a decisive role in supplying active ingredients as practical products rather than just products in a iontophoresis concept.

### [Electromotive Force Experiments When Patches Are Attached to Skin depending on Presence and Length of Micro-needles]

Figures 11 (a) to (c) show photographs of self-current-generating skin-applicable patch prototypes containing a biodegradable metal according to the present disclosure, respectively, showing cases without micro-needles, with 100 µm-long needles formed, and with 230 µm-long needles formed.

As shown in the photographs, a first electrode layer, the entire area of which was in contact with the skin, was a 0.07 mm thick magnesium metal sheet with a plurality of hole-shaped penetration parts, a second electrode layer, which was partially in contact with the skin, was a 0.07 mm copper metal sheet, and a 0.28 mm pure cotton sheet was used as the support layer for the ionic solution.

The first electrode layer and the second electrode layer were electrically insulated and spaced apart by a pure cotton sheet, with no direct electrical connection. 0.9% NaCl aqueous solution was used as an electrolyte for the ionic solution.

In this state, each of the patches shown in Figure 11 was applied to the user's skin, and the operating voltage and operating current were measured. The results are shown in Table 4 below.

**[Table 4]**

| Patch | V_{L} [V] | I_{L} [mA] | P_{L} [µW] |
|---|---|---|---|
| (a) | 1.487 | 0.115 | 0.171 |
| (b) | 1.417 | 0.326 | 0.461 |
| (c) | 1.434 | 1.935 | 2.774 |

That is, it was confirmed, compared to the patch without micro-needles in (a), the patches in (b) and (c) showed a significant increase in electromotive force as the amount of current increased. In particular, a nearly 6 times increase in electromotive force was confirmed in (c) compared to (b). Through this, it was estimated that when the length of the micro-needles exceeds 100 µm or more and penetrates the stratum corneum and epidermis of the skin to reach the dermis, a dramatic reduction in skin resistance could be achieved. Therefore, it was preferable that the thickness of the micro-needles was at least 50 µm or more, which was the typical skin thickness.

Furthermore, as applied to the preparation of the patches in this experiments, the thinner the thickness of a thin plate in the first or second area, the better the adhesion and attachment of the patches to the skin, so it was desirable that the thickness of the micro-needles be within an appropriate range. In particular, in the case of the biodegradable metal according to the present disclosure, it was not easy to process thin plates with a thickness of less than 0.07 mm, and when the thickness was 0.7 mm or more, a phenomenon of lifting from the skin was confirmed despite the ductility of the metal. Therefore, the thickness range was preferably 0.07 to 0.7 mm.

Meanwhile, through various experiments, it was confirmed that the larger the volume of the support layer 30 supporting the ionic solution, that is, the larger the amount of electrolyte solution, the greater the electrical physical quantity. However, according to the embodiments of the present disclosure, in the case of a patch product having a structure in which the support layer was interposed between the first electrode layer and the second electrode layer, there was a limitation on increasing the thickness of the support layer. Therefore, it was desirable to make the area of the support layer as wide as possible. Accordingly, it was preferable that the first area 10 corresponding to the area of the support layer 30 had more than twice as large an area as the second area 20.

In this regard, the sixth embodiment shown in Figure 14 will be described as another modified example of the third embodiment, the basic type.

Figure 14 shows an exploded perspective view showing a structure in which the first area 10 and the second area 20, which are the skin-contacting sides, are arranged at the upper part in the drawing, and the second electrode layer 22 and the support layer 30 are sequentially arranged at the lower part in a laminating order.

That is, in the structure, the first area 10 and the second area 20 might be made of a biodegradable metal having a magnesium-based composition according to Formula 1, which will be described later. Meanwhile, the second area 20 was bonded to be electrically connected to the material forming the second electrode layer 22. With that, the first area was coupled to the second electrode layer 22 through an insulating layer 60 so as not to be in direct electrical contact.

In other words, this integrated structure of the insulating layer 60 and the second electrode layer 22 was formed by coating a conductive material on one side of an insulating film (PET film) to form the second electrode layer 22 and then removing the insulating film in some regions. Alternatively, such an integrated structure was formed by including conductive fibers (copper fibers and silver fibers). As a result, such an integrated structure might be formed in a way that allows the first area 10 to be laminated and bonded on the insulating layer 60, which was formed by combining an insulating bonding means with a partial region of one side of a conductive cloth.

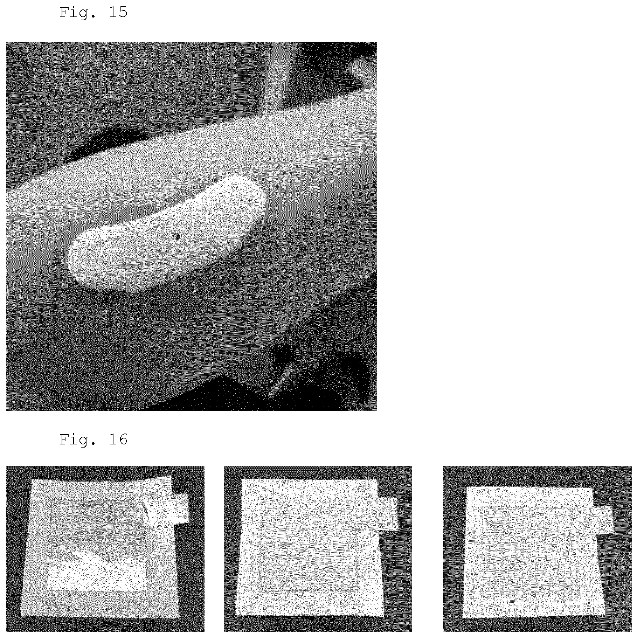

This means that the formation of the structure shown in Figure 14 might be implemented by cutting silver cloth woven with silver fibers into the corresponding shape as a material for the second electrode layer 22, and then applying an insulating adhesive or an insulating tape to some regions on one side of the cloth to form the insulating layer 60 shown in white in Figure 15, followed by bringing a biodegradable metal corresponding to the second area 20 to be bonded to a black portion where the material surface of the second electrode layer 22 was exposed while bringing a biodegradable metal for the first electrode layer 11 corresponding to the first area 10 to be bonded to a white portion where the second electrode layer 22 was insulated.

Meanwhile, unlike the previous embodiments in which the support layer 30 was structured to be interposed between the first electrode layer 11 and the second electrode layer 22, in the embodiment illustrated in Figure 14, the support layer 30 was located outside the second electrode layer 22 (the bottom in the drawing, opposite to the skin attachment surface). Thereby, a structure in which the support layer 30 was located on the outermost surface might be formed when a patch is attached to the skin and used.

This structure was formed by first laminating the first electrode layer 11 and the second electrode layer 22 in the form of a thin film and then bonding the support layer, such as a natural pulp sheet or rayon sheet, to the outside, which was compared to a structure where a support layer 30 is interposed between the first and second electrode layers. Therefore, the structure not only became easier to form but was also advantageous in securing a region for the support layer 30, and showed excellent robustness in the commercialized state.

Moreover, in a structure in which there was no support layer for the ionic solution between the first electrode layer 11 and the second electrode layer 22, as in the corresponding structure, to initiate self-current generation in the state where the patch was attached without prior supply of ionic solution, the second electrode layer 22 was also required to be structured to be open toward the top by having an opening 65. For this, the second electrode layer 22 was preferably provided with an opening 65. Through this, the ionic solution might be easily and continuously supplied to the first area 10 and the skin below the first area 10 without limitation in the amount supplied.

At this time, the support layer 30 was further combined in a form of covering at least part (preferably all) of the opening 65. Thereby, the ionic solution supplied from the top instantly spread across the entire area of the support layer 30, which might facilitate electrochemical reactions more smoothly and allowed the supported ionic solution to be supplied slowly and consistently to the inside of the patch. Through this, ion exchange might occur between the first electrode layer 11 and the exposed portion of the electrically insulated second electrode layer 22 by the electrolytic action of the ionic solution.

By a patch being configured in this way, in the structure in which the support layer 30 was fixedly interposed between the first electrode layer 11 and the second electrode layer 22, as in the first to fourth embodiments, problems, including the limited area of the support layer 30, skin adhesion weakening caused by moisture leaking from the support layer 30 due to the position thereof inside the patch, and difficulty in spreading of the ionic solution across the entire surface of the first electrode layer 11 might be solved. In addition, additional benefits might be expected, such as acting as a filter capable of filtering out foreign substances mixed in an ionic solution.

Figure 15 shows a photograph showing a patch of the sixth embodiment actually implemented and attached to the skin. By being configured in this way, it was confirmed that the self-current-generating skin-applicable patch containing a biodegradable metal according to the present disclosure might be realized in a structure that can smoothly and continuously supply an ionic solution containing active ingredients through the uppermost support layer 30 even when the patch was attached to the skin.

### [Evaluation of Characteristics of Patches containing Ag/AgCl Composite Electrode Materials]

After the electromotive force experiments using a combination of the previously confirmed magnesium metal as the first electrode layer and the previously confirmed Cu or SUS301 as the second electrode layer, characteristics of patch structures that were easy to realize in the same structure as the embodiment 6 described above were also evaluated. As the first electrode layer, a 0.07 mm thick magnesium metal sheet containing inevitable impurities was prepared. As the second electrode layer, two types of electrode layers were prepared by coating a paste-like Ag/AgCl composite on a 0.05 mm thick PET film. As a comparison sample, a Cu thin plate with a thickness of 0.03 mm was prepared. Finally, three types of samples were prepared in which the first electrode layer and each of the second electrode layers were laminated and bonded in an insulated state. The shape of the samples is shown in Figure 16, and it was confirmed that they had the characteristics according to Table 5 below in order from the left.

**[Table 5]**

| Sample | 1 | 2 | 3 |
|---|---|---|---|
| Material | Cu | Ag/AgCl | Ag/AgCl |
| Form | Foil | Printed Film | Printed Film |
| Thickness [µm] | 30 | 65 (Coating) + 50 (PET) | 130 (Coating) + 50 (PET) |
| Electrical Resistance [Ω*cm] | 0.15 | 0.40 | 0.29 |
| Remarks | Reference | Doctor blade and heat-treatment | |

The paste-like Ag/AgCl composite used in the experiments was SC 141 sold by Keti Co., Ltd., and was cured by heating at a temperature of 120°C for 30 minutes. Accordingly, the experiments were conducted by coating the Ag/AgCl composite on a 50 µm PET film at different thicknesses as follows. The results of the experiments with the samples under a load resistance of 500 Ω are summarized in Table 6 below. The applied electrolyte solution was 0.9% NaCl aqueous solution.

**[Table 6]**

| Condition | Sample | 1 | 2 | 3 |
|---|---|---|---|---|
| 500 Ω | V_{L} [V] | 0.694 | 1.225 | 1.365 |
| | I_{L} [mA] | 1.356 | 2.410 | 2.727 |
| | P_{L} [mW] | 0.941 | 2.952 | 3.722 |

Compared to Sample 1, which was the result of an experiment using a Cu thin plate, the cases of Ag/AgCl composite more clearly showed increases in operating voltage, operating current, and electromotive force. Through the results of samples 2 and 3, it was confirmed that the operating voltage, operating current, and electromotive force increased proportionally as the Ag/AgCl composite coating layer became thicker. When the second electrode layer + insulating layer structure was realized in the form of a film-like coating layer, it was confirmed that there was an appropriate thickness in terms of crumbling and flexibility. In conclusion, it was confirmed that there is an electrical advantage in using Ag as a material for the second electrode layer compared to Cu. In addition, it was confirmed that a product that was advantageous in terms of patch flexibility and adhesion could be prepared as the second electrode layer by using silver cloth woven with silver fibers rather than coating.

Although specific embodiments according to the present disclosure have been described so far, it is obvious that various modifications are possible without departing from the scope of the present disclosure.

Therefore, the scope of the present disclosure should not be limited to the described embodiments but should be determined by the scope of the patent claims described later as well as the equivalents to the claims of this patent.

## Claims

1. A self-current-generating skin-applicable patch, comprising:
a first area (10) in contact with an area of the user's skin and
a second area (20) being spaced apart from the first area (10) and in contact with another area of the skin adjacent to the area of the skin where the first area (10) is in contact,
wherein the first area (10) or the second area (20) comprises a biodegradable metal which can be decomposed and absorbed by coming into contact with the user's skin or reacting with moisture within the tissue,
wherein the patch comprises:
a first electrode layer (11), which is provided in the form of a thin sheet in direct contact with the user's skin as a structure integrated with the first area (10) and has at least one penetration part fluid-transferable from the side opposite to a skin-contacting side,
a second electrode layer (22), which is made of a different material having a standard reduction potential that differs from that of a material forming the first electrode layer (11), is positioned at the side opposite to the skin-contacting side of the first electrode layer (11) while being insulated from the first electrode layer (11), and is at least partially extended so as to be electrically connected to the second area (20), and
a support layer (30), which is in the form of a sheet having a thickness and is positioned at the side opposite to the skin-contacting side of the first electrode layer (11), and is made of a hydrophilic material capable of supporting an ionic solution.

2. The patch of Claim 1, wherein the first area (10) or the second area (20) comprises at least one micro-needle (15) protruding in the direction of contact with the user's skin.

3. The patch of any one of Claims 1 or 2, wherein the biodegradable metal is represented by the Formula 1 below,
[Formula 1] MgₐZn_{b}Ca_{c}X_{d}
wherein, in Formula 1, a, b, c and d represent the weight percentages of each component, a+b+c+d = 100 weight%, a is the largest, b, c, and d satisfy the ranges of 0≤b≤5, 0≤c≤1, 0≤d≤1, and X is composed of at least one type of impurities consisting of elements other than Mg, Zn, and Ca.

4. The patch of Claim 3, wherein the first area (10) and the first electrode layer (11) comprise a biodegradable metal according to Formula 1 above, and
the second electrode layer (22) comprises a different material with a higher standard reduction potential than the material of the first electrode layer (11).

5. The patch of Claim 4, wherein the second electrode layer (22) comprises at least one material selected from the group consisting of copper, zinc, silver, silver chloride, iron, and stainless steel.

6. The patch of Claim 5, wherein the second area (20) comprises a biodegradable metal according to Formula 1 and is configured to be electrically connected to a portion extending from the second electrode layer (22).

7. The patch of Claim 3, wherein the second electrode layer (22) comprises a biodegradable metal according to Formula 1, and
the first electrode layer (11) is made of a heterogeneous material with a higher standard reduction potential than the material of the second electrode layer (22).

8. The patch of Claim 2, wherein the micro-needle (15) have a length of 0.02 mm or more.

9. The patch of Claim 1, wherein the support layer (30) is positioned in a laminated form by being interposed between the first electrode layer (11) and the second electrode layer (22) .

10. The patch of Claim 1, wherein the first electrode layer (11) and the second electrode layer (22) are laminated while being insulated from each other by an insulating layer (60),
Wherein the second electrode layer (22) is provided with an opening (65) that exposes at least a portion of the penetration part of the first electrode layer (11), and
the support layer (30) covers at least a portion of the opening (65) on the side opposite to the skin-contacting side of the first electrode layer (11).

11. The patch of Claim 2, wherein the penetration part formed in the first electrode layer (11) has a perforation in the form of a hole, and
at least one micro-needle (15) is bent along the edge of the hole in the direction of contact with the user's skin.

12. The patch of Claim 4, wherein the second electrode layer (22) is applied in the form of a flexible cloth made of conductive fiber.

13. The patch of Claim 1, wherein the ionic solution comprises at least one type selected from the group consisting of phosphate-buffered saline and sodium chloride aqueous solution.

14. The patch of any one of Claims 1 or 13, wherein the ionic solution comprises at least one type of active ingredients for functional cosmetics and pharmaceuticals.

15. The patch of Claim 14, wherein the active ingredients are ionic.

16. The patch of Claim 1, wherein the support layer (30) is made of at least one type selected from the group consisting of pure cotton sheets, natural pulp sheets, and rayon sheets, which are dried fabric-type materials.

17. The patch of any one of Claims 1 or 16, wherein the support layer (30) further comprises a capsule, which discharges the ionic solution by the user's initiating action.

18. The patch of Claim 9, wherein the first area (10) has at least twice as large an area as the second area (20).

19. The patch of Claim 1, wherein the first area (10) formed integrally with the first electrode layer (11) has a thin sheet shape and a thickness of 0.07 to 0.7 mm.
